(19) Europäisches Patentamt
European Patent Office
Office européen des brevets

(11) **EP 4 563 024 A1**

(12) **EUROPEAN PATENT APPLICATION**
published in accordance with Art. 153(4) EPC

(43) Date of publication:
**04.06.2025   Bulletin 2025/23**

(21) Application number: **22953096.9**

(22) Date of filing: **28.07.2022**

(51) International Patent Classification (IPC):
***A24F 40/57*** *(2020.01)*

(52) Cooperative Patent Classification (CPC):
**A24F 40/57**

(86) International application number:
**PCT/JP2022/029039**

(87) International publication number:
**WO 2024/024003 (01.02.2024 Gazette 2024/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Japan Tobacco Inc.
Tokyo 105-6927 (JP)**

(72) Inventors:
• **YAMADA, Kentaro
Tokyo 130-8603 (JP)**
• **NAGAHAMA, Toru
Tokyo 130-8603 (JP)**

(74) Representative: **Hoffmann Eitle
Patent- und Rechtsanwälte PartmbB
Arabellastraße 30
81925 München (DE)**

(54) **AEROSOL GENERATION SYSTEM, CONTROL METHOD, AND PROGRAM**

(57)    [Problem] To provide a mechanism capable of further improving heating efficiency.

[Solution] An aerosol generation system comprising: a power supply unit; a heating unit that heats an aerosol source contained in a base material using power supplied from the power supply unit; and a control unit that controls the operation of the heating unit, wherein the control unit executes output control so as to increase the voltage applied to the heating unit per unit time with an increase in the temperature of the heating unit.

FIG. 1

EP 4 563 024 A1

## Description

Technical Field

The present disclosure relates to an aerosol generation system, a control method, and a program.

Background Art

Inhaler devices that generate a substance to be inhaled by users, such as electronic cigarettes and nebulizers, are widely used. An inhaler device generates an aerosol with a flavor component, for example, using a substrate including an aerosol source for generating an aerosol and a flavor source for imparting a flavor component to the generated aerosol. A user can taste a flavor by inhaling the aerosol with the flavor component generated by the inhaler device. A flavor tasted by a user will also be referred to as a smoke taste. Inhalation of an aerosol by a user will be referred to as a puff or a puff action hereinafter.

Numerous techniques relating to a process for heating a substrate have been developed so far. The following Patent Literature 1, for example, discloses a technique for determining a duty ratio in PWM (pulse width modulation) on the basis of information regarding a battery at a start of heating.

Citation List

Patent Literature

Patent Literature 1: JP 6930689 B2

Summary of Invention

Technical Problem

The technique disclosed in Patent Literature 1, however, has room for improvement in heating efficiency.

The present disclosure, therefore, has been conceived in view of the above problem, and an object thereof is to provide a mechanism capable of further improving heating efficiency.

Solution to Problem

In order to solve the above problem, according to an aspect of the present invention, an aerosol generation system including a power supply, a heater that heats an aerosol source included in a substrate using power supplied from the power supply, and a controller that controls operation of the heater is provided. The controller performs output control for increasing a voltage applied to the heater in unit time as temperature of the heater increases.

The aerosol generation system may further include a first switching element that starts or stops the supply of power to the heater. The controller may control, as the output control, the first switching element such that a period of time for which power is supplied to the heater in unit time becomes longer as the temperature of the heater increases.

The aerosol generation system may further include a plurality of transformers that is provided between the power supply and the heater and that transforms a voltage applied from the power supply and applies resultant voltages to the heater and a second switching element that switches the one transformer that applies a voltage to the heater between the plurality of transformers. Output voltages of the plurality of transformers may be different from each other. The controller may control, as the output control, the second switching element such that the transformer whose output voltage is higher applies a voltage to the heater as the temperature of the heater increases.

The controller may perform the output control in a period after a start of the heating in which the temperature of the heater continues to increase.

The heater may be a resistance heater whose resistance changes in accordance with the temperature thereof. The controller may perform the output control such that an output of the heater becomes a predetermined target value.

The controller may set the target value on a basis of a maximum output of the power supply.

The controller may decrease the voltage applied to the heater in unit time as the temperature of the heater at a start of the heating increases.

The controller may control the first switching element such that the period of time for which power is supplied to the heater in unit time becomes shorter as the temperature of the heater at a start of the heating increases.

The controller may control the second switching element such that the transformer whose output voltage is lower applies a voltage to the heater as the temperature of the heater at a start of heating increases.

The controller may decrease the target value as the temperature of the heater at a start of the heating increases.

The controller may control a timing of performing the output control on a basis of a value corresponding to the temperature of the heater.

The controller may control a timing of performing the output control on a basis of time elapsed since a start of the heating.

The aerosol generation system may further include the substrate.

In addition, in order to solve the above problem, according to another aspect of the present invention, a control method executed by a computer that controls an inhaler device is provided. The inhaler device includes a power supply and a heater that heats an aerosol source included in a substrate using power supplied from the power supply. The control method includes performing output control for increasing a voltage applied to the heater in unit time as temperature of the heater increases.

In addition, in order to solve the above problem, according to another aspect of the present invention, a program executed by a computer that controls an inhaler device is provided. The inhaler device includes a power supply and a heater that heats an aerosol source included in a substrate using power supplied from the power supply. The program causes the computer to perform a process including performing output control for increasing a voltage applied to the heater in unit time as temperature of the heater increases.

Advantageous Effects of Invention

As described above, according to the present disclosure, a mechanism capable of further improving heating efficiency is provided.

Brief Description of Drawings

[Fig. 1] Fig. 1 is a schematic diagram schematically illustrating a configuration example of an inhaler device.

[Fig. 2] Fig. 2 is a graph illustrating an example of changes in temperature of a heater in a case where temperature control is performed on the basis of a heating profile shown in Table 1.

[Fig. 3] Fig. 3 is a block diagram for describing output control according to an embodiment of the present disclosure.

[Fig. 4] Fig. 4 is a graph for describing the output control according to the embodiment.

[Fig. 5] Fig. 5 is a flowchart illustrating an example of a procedure of a process performed by an inhaler device according to the embodiment.

[Fig. 6] Fig. 6 is a block diagram for describing output control according to a first modification.

[Fig. 7] Fig. 7 is a flowchart illustrating an example of a procedure of a process performed by an inhaler device according to the modification.

Description of Embodiments

A preferred embodiment of the present disclosure will be described in detail hereinafter with reference to the accompanying drawings. Structural elements having substantially the same functional configuration will be given the same reference numerals herein and in the drawings, and redundant description thereof is omitted.

<1. Configuration example>

An inhaler device generates material to be inhaled by a user. In the example described below, the material generated by the inhaler device is an aerosol. Alternatively, the material generated by the inhaler device may be gas.

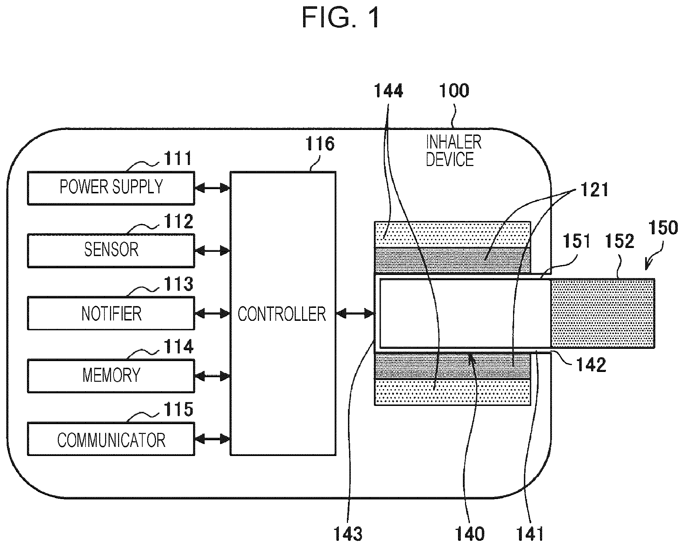

Fig. 1 is a schematic diagram of the inhaler device according to the configuration example. As illustrated in Fig. 1, an inhaler device 100 according to the present configuration example includes a power supply 111, a sensor 112, a notifier 113, a memory 114, a communicator 115, a controller 116, a heater 121, , a container 140, and a heat insulator 144.

The power supply 111 stores electric power. The power supply 111 supplies electric power to the structural elements of the inhaler device 100 under the control of the controller 116. The power supply 111 may be a rechargeable battery such as a lithium ion secondary battery.

The sensor 112 acquires various items of information regarding the inhaler device 100. In an example, the sensor 112 may be a pressure sensor such as a condenser microphone, a flow sensor, or a temperature sensor, and acquire a value generated in accordance with the user's inhalation. In another example, the sensor 112 may be an input device that receives information input by the user, such as a button or a switch.

The notifier 113 provides information to the user. The notifier 113 may be a light-emitting device that emits light, a display device that displays an image, a sound output device that outputs sound, or a vibration device that vibrates.

The memory 114 stores various items of information for operation of the inhaler device 100. The memory 114 may be a non-volatile storage medium such as flash memory.

The communicator 115 is a communication interface capable of communication in conformity with any wired or wireless communication standard. Such a communication standard may be, for example, Wi-Fi (registered trademark), Bluetooth (registered trademark), Bluetooth Low Energy (BLE) (registered trademark), near-field communication (NFC), or a standard using a low power wide area (LPWA).

The controller 116 functions as an arithmetic processing unit and a control circuit, and controls the overall operations of the inhaler device 100 in accordance with various programs. The controller 116 includes an electronic circuit such as a central processing unit (CPU) or a microprocessor, for example.

The container 140 has an internal space 141, and holds a stick substrate 150 in a manner partially accommodated in the internal space 141. The container 140 has an opening 142 that allows the internal space 141 to communicate with outside. The container 140 accommodates the stick substrate 150 that is inserted into the internal space 141 through the opening 142. For example, the container 140 may be a tubular body having the opening 142 and a bottom 143 on its ends, and may define the pillar-shaped internal space 141. The container 140 connects with an airflow path that supplies air to the internal space 141. For example, a side surface of the inhaler device 100 has an air inlet hole that is an inlet of air into the airflow path. For example, the bottom 143 has an air outlet hole that is an outlet of the air from the airflow path to the internal space 141.

The stick substrate 150 includes a substrate 151 and an inhalation port 152. The substrate 151 includes an aerosol source. The aerosol source includes a flavor component that is either derived from tobacco or not derived from tobacco. For the inhaler device 100 that is a medical inhaler such as a nebulizer, the aerosol source may include a medicine. For example, the aerosol source may be a liquid including the flavor component that is either derived from tobacco or not derived from tobacco, such as polyhydric alcohol or water. Examples of the polyhydric alcohol include glycerine and propylene glycol. Alternatively, the aerosol source may be a solid including the flavor component that is either derived from tobacco or not derived from tobacco. The stick substrate 150 held by the container 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 through the airflow path (not illustrated), and the air and an aerosol generated from the substrate 151 reach inside the mouth of the user.

The heater 121 heats the aerosol source to atomize the aerosol source and generate the aerosol. In the example illustrated in Fig. 1, the heater 121 has a film-like shape and surrounds the outer circumference of the container 140. Subsequently, heat produced from the heater 121 heats the substrate 151 of the stick substrate 150 from the outer circumference, generating the aerosol. The heater 121 produces heat when receiving electric power from the power supply 111. In an example, the electric power may be supplied in response to the sensor 112 detecting a start of the user's inhalation and/or an input of predetermined information. Subsequently, the supply of the electric power may be stopped in response to the sensor 112 detecting an end of the user's inhalation and/or an input of predetermined information.

The heat insulator 144 prevents heat from transferring from the heater 121 to the other structural elements. For example, the heat insulator 144 may be a vacuum heat insulator or an aerogel heat insulator.

The configuration example of the inhaler device 100 has been described above. The inhaler device 100 is not limited to the above configuration, and may be configured in various ways as exemplified below.

In an example, the heater 121 may have a blade-like shape, and may be disposed so that the heater 121 protrudes from the bottom 143 of the container 140 toward the internal space 141. In this case, the heater 121 having the blade-like shape is inserted into the substrate 151 of the stick substrate 150 and heats the substrate 151 of the stick substrate 150 from its inside. In another example, the heater 121 may be disposed so that the heater 121 covers the bottom 143 of the container 140. In still another example, the heater 121 may be implemented as a combination of two or more selected from a first heater that covers the outer circumference of the container 140, a second heater having the blade-like shape, and a third heater that covers the bottom 143 of the container 140.

In another example, the container 140 may include an opening/closing mechanism that at least partially opens and closes an outer shell defining the internal space 141. Examples of the opening/closing mechanism include a hinge. In addition, the container 140 may accommodate the stick substrate 150 while sandwiching the stick substrate 150 inserted into the internal space 141 by opening and closing the outer shell. In this case, the heater 121 may be at the sandwiching position of the container 140 and may produce heat while pressing the stick substrate 150.

In addition, means for atomizing the aerosol source is not limited to heating by the heater 121. For example, the means for atomizing the aerosol source may be induction heating. In this case, the inhaler device 100 includes at least an electromagnetic induction source such as a coil that generates a magnetic field instead of the heater 121. The inhaler device 100 may be provided with a susceptor that produces heat by the induction heating, or the stick substrate 150 may include the susceptor.

The inhaler device 100 operates together with the stick substrate 150 to generate the aerosol to be inhaled by the user. A combination of the inhaler device 100 and the stick substrate 150, therefore, may be regarded as an aerosol generation system.

<2. Technical features>

(1) Heating profile

The controller 116 controls the operation of the heater 121 on the basis of a heating profile. The control of the operation of the heater 121 is achieved by controlling the supply of power from the power supply 111 to the heater 121. The heater 121 heats the stick substrate 150 (more specifically, the aerosol source included in the stick substrate 150) using the power supplied from the power supply 111.

The heating profile is control information for controlling heating temperature of the aerosol source. The heating profile may be control information for controlling the temperature of the heater 121. In an example, the heating profile can include a target value of the heating temperature (hereinafter also referred to as a target temperature) of the aerosol source. The target temperature may change in accordance with time elapsed since a start of heating, and in this case, the heating profile includes information that defines temporal changes in the target temperature. In another example, the heating profile can include a parameter that defines a method for supplying power to the heater 121 (hereinafter also referred to as a power supply parameter). The power supply parameter includes, for example, a voltage applied to the heater 121, on/off of the supply of power to the heater 121, a feedback control method to be employed, or the like. On/off of the supply of power to the heater 121 may be regarded as on/off of the heater 121.

The controller 116 controls the operation of the heater 121 such that the temperature (hereinafter also referred as an actual temperature) of the heater 121 changes in the same manner as the target temperature defined in the heating profile. The heating profile is typically designed in such a way as to optimize the flavor tasted by the user when the user inhales the aerosol generated from the stick substrate 150. By controlling the operation of the heater 121 on the basis of the heating profile, therefore, the flavor tasted by the user can be optimized.

The control of the temperature of the heater 121 can be achieved through, for example, known feedback control. The feedback control may be, for example, a PID (proportional-integral-differential) controller. The controller 116 can supply the power from the power supply 111 to the heater 121 in a form of a pulse based on pulse width modulation (PWM) or pulse frequency modulation (PFM). In this case, the controller 116 can control the temperature of the heater 121 by adjusting a duty ratio of the power pulse in the feedback control. Alternatively, the controller 116 may perform simple on/off control in the feedback control. For example, the controller 116 may cause the heater 121 to produce heat until the actual temperature reaches the target temperature, cause the heater 121 to stop producing heat when the actual temperature reaches the target temperature, and cause the heater 121 to resume the heating when the actual temperature falls below the target temperature.

In an example, the temperature of the heater 121 can be quantified by measuring or estimating resistance (more specifically, electrical resistance) of the heater 121 (more specifically, a resistance heater constituting the heater 121). This is because the resistance of the resistance heater changes in accordance with the temperature. The resistance of the resistance heater can be estimated by measuring a decrease in voltage of the resistance heater. The decrease in the voltage of the resistance heater can be measured by a voltage sensor that measures a potential difference applied to the resistance heater. Alternatively, the temperature of the heater 121 may be measured by a thermistor provided near the heater 121. A thermistor is a resistor whose resistance changes in accordance with temperature.

A period of time from a start to an end of a process for generating the aerosol using the stick substrate 150 will be referred to as a heating session hereinafter. In other words, a heating session is a period of time in which the operation of the heater 121 is controlled on the basis of a heating profile. A start of a heating session is a time when heating based on a heating profile starts. An end of a heating session is a time when a sufficient amount of aerosol is no longer generated. A heating session includes a preheating period and a puffable period following the preheating period. The puffable period is a period when a sufficient amount of aerosol is assumed to be generated. The preheating period is a period from a start of heating until the puffable period starts. Heating performed in the preheating period will also be referred to as preheating.

The following Table 1 shows an example of the heating profile.

[Table 1]

Table 1. Example of heating profile

| Period | | Temporal changes in target temperature | Temporal changes in power supply parameter |
|--------|--|--|--|
| Section | Duration | | |
| STEP 0 | - | Increase to 300°C (no time control) | ON |
| STEP 1 | 10 sec. | Maintain at 300°C | ON |
| STEP 2 | - | Decrease to 220°C (no time control) | OFF |
| STEP 3 | - | Increase to 230°C (no time control) | ON |
| STEP 4 | 60 sec. | Maintain at 230°C | ON |
| STEP 5 | 60 sec. | Increase to 260°C | ON |
| STEP 6 | 60 sec. | Maintain at 260°C | ON |
| STEP 7 | 5 sec. | - | OFF |

As shown in Table 1, the heating profile may be divided into a plurality of periods, and a temporal change in the target temperature and a temporal change in the power supply parameter may be specified for each period. In the example shown in Table 1, the heating profile is divided into a total of eight periods of STEP 0 to STEP 7. For each step, a temporal change in the target temperature and a temporal change in the power supply parameter are specified. The steps specified in the heating profile are examples of a unit period in the present embodiment.

In each step, time control might be performed. The time control is control for ending the step when a predetermined period of time (that is, duration set for each step) elapses. When the time control is performed, a rate of change in the temperature of the heater 121 may be controlled such that the temperature of the heater 121 reaches the target temperature at an end of the duration. In addition, when the time control is performed, the temperature of the heater 121 may be controlled such that the temperature of the heater 121 reaches the target temperature halfway through the duration and remains at the target temperature until the end of the duration. In the example shown in Table 1, the time control is performed in STEP 1 and STEP 4 to STEP 7. A period in which the time control is performed will also be referred to as a time-fixed period hereinafter.

In each step, the time control might not be performed. When the time control is not performed, the step ends when the temperature of the heater 121 reaches a predetermined temperature (that is, the target temperature set for each step). The duration of a step in which the time control is not performed, therefore, changes in accordance with a rate of change in temperature. In the example shown in Table 1, the time control is not performed in STEPs 0, 2, and 3. A period in which the time control is not performed will also be referred to as a time-variable period hereinafter.

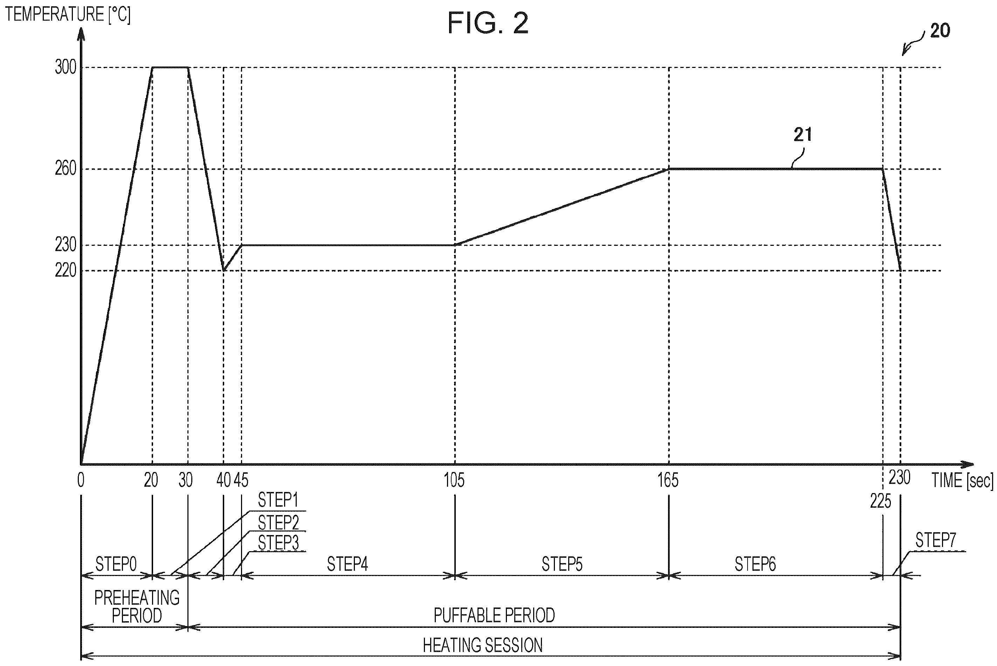

Changes in the temperature of the heater 121 when the controller 116 performs the temperature control in accordance with the heating profile shown in Table 1 will be described with reference to Fig. 2. Fig. 2 is a graph illustrating an example of changes in the temperature of the heater 121 at a time when the temperature control is performed on the basis of the heating profile shown in Table 1. A horizontal axis of a graph 20 represents time (seconds). A vertical axis of the graph 20 represents the temperature of the heater 121. A line 21 indicates the changes in the temperature of the heater 121. As illustrated in Fig. 2, the temperature of the heater 121 changes in the same manner as the target temperature specified in the heating profile. An example of the heating profile will be described hereinafter with reference to Table 1 and Fig. 2.

As shown in Table 1 and Fig. 2, in STEP 0, the temperature of the heater 121 increases to 300°C from an initial temperature. The initial temperature is the temperature of the heater 121 at a start of heating. In STEP 0, the time control is not performed. STEP 0, therefore, ends when the temperature of the heater 121 reaches 300°C. In the example illustrated in Fig. 2, STEP 0 ends in 20 seconds. Thereafter, in STEP 1, the temperature of the heater 121 is maintained at 300°C. The preheating period ends as STEP 1 ends, and the puffable period starts as STEP 2 starts.

It is desirable for the user that the preheating period is short. If the stick substrate 150 is not sufficiently heated, however, moisture that has not been fully evaporated might remain inside the stick substrate 150. If the user puffs in this state, hot steam might be delivered to the inside of the user's mouth. It is therefore desirable for the preheating period to last for a certain length of time. In an example, it is desirable to sharply increase the temperature of the heater 121 to 300°C in STEP 0 and to secure a certain duration of STEP 1.

As shown in Table 1 and Fig. 2, in STEP 2, the temperature of the heater 121 decreases to 220°C. In STEP 2, the time control is not performed. STEP 2, therefore, ends when the temperature of the heater 121 reaches 220°C. In the example illustrated in Fig. 2, STEP 2 ends in 10 seconds. In STEP 2, the supply of power to the heater 121 is stopped. It is therefore possible to decrease the temperature of the heater 121 as fast as possible. By decreasing the temperature of the heater 121 halfway through the heating session like this, rapid consumption of the aerosol source can be prevented. As a result, it is possible to prevent depletion of the aerosol source during the heating session.

As shown in Table 1 and Fig. 2, next, in STEP 3, the temperature of the heater 121 increases to 230°C. In STEP 3, the time control is not performed. STEP 3, therefore, ends when the temperature of the heater 121 reaches 230°C. In the example illustrated in Fig. 2, STEP 3 ends in 5 seconds. By providing a period in which the temperature of the heater 121 is increased again after being decreased, an excessive drop in the temperature of the heater 121 can be prevented.

As shown in Table 1 and Fig. 2, next, in STEP 4 to STEP 6, the temperature of the heater 121 is increased to 260°C stepwise. By gradually increasing the temperature of the heater 121 like this, it is possible to maintain the amount of aerosol generated while suppressing power consumption in the entirety of the heating session.

As shown in Table 1 and Fig. 2, in STEP 7, the temperature of the heater 121 decreases. In STEP 7, the supply of power to the heater 121 is stopped. In STEP 7, the duration is specified, but the target temperature is not specified. STEP 7, therefore, ends at an end of the duration. In STEP 7, a sufficient amount of aerosol can be generated because of residual heat in the stick substrate 150. In this example, therefore, the puffable period, that is, the heating session, ends as STEP 7 ends.

The notifier 113 may notify the user of information indicating a timing at which the preheating ends. For example, the notifier 113 notifies of information for informing the user of the end of the preheating before the preheating ends or, after the preheating ends, notifies of information indicating that the preheating has ended. The notification for the user can be performed, for example, by turning on an LED, performing vibration, or the like. The user can puff immediately after the end of the preheating on the basis of such notification.

Similarly, the notifier 113 may notify the user of information indicating a timing at which the puffable period ends. For example, the notifier 113 notifies of information for informing the user of the end of the puffable period before the puffable period ends or, after the puffable period ends, notifies of information indicating that the puffable period has ended. The notification for the user can be performed, for example, by turning on the LED, performing vibration, or the like. The user can puff until the puffable period ends on the basis of such notification.

The above-described heating profile is merely an example, and various other examples are conceivable. For example, the number of steps, the duration and the target temperature of each step may be changed appropriately.

(2) Output control in preheating

The heater 121 is configured as a resistance heater. The resistance of the heater 121 (more specifically, the resistance heater constituting the heater 121) changes as the temperature of the heater 121 changes. In particular, as the temperature of the heater 121 increases, the resistance of the heater 121 increases. When the voltage applied to the heater 121 is fixed, heating efficiency decreases as the resistance of the heater 121 increases.

The controller 116, therefore, performs output control of the heater 121. The output control of the heater 121 is control for increasing the voltage applied to the heater 121 in unit time as the temperature of the heater 121 increases. More specifically, the controller 116 increases the voltage applied to the heater 121 in unit time in accordance with an increase in the resistance of the heater 121, which accompanies an increase in the temperature of the heater 121. With this configuration, a decrease in the heating efficiency due to an increase in the resistance of the heater 121 can be cancelled by increasing the voltage applied to the heater 121 in unit time. That is, the heating efficiency can be kept high.

The controller 116 performs the output control in a period in which the temperature of the heater 121 continues to increase after heating by the heater 121 starts. More specifically, the controller 116 performs the output control in the preheating period, or more specifically, in the time-variable period in the preheating period (e.g., STEP 0 in the example shown in Table 1 and Fig. 2). With this configuration, a desired level of heating efficiency can be maintained in the preheating period, and the length of the preheating period can be optimized. As a result, the preheating period can be shortened, for example, and usability improves.

The controller 116 performs the output control such that an output of the heater 121 becomes a predetermined target value. More specifically, the controller 116 performs the output control such that the output of the heater 121 calculated on the basis of the resistance of the heater 121 and the voltage applied to the heater 121 becomes the predetermined target value (hereinafter also referred to as an output target). With this configuration, since the output of the heater 121 becomes constant at or near the output target, a load of the power supply 111 becomes constant. As a result, deterioration of the power supply 111 can be reduced.

The controller 116 may set the output target on the basis of a maximum output of the power supply 111. For example, the controller 116 may set a value substantially equal to the maximum output as the output target by, for example, setting the output target to about 90% of the maximum output of the power supply 111. With this configuration, the heating efficiency of the heater 121 can be maximized.

The controller 116 may control a timing at which the output control is performed on the basis of a value corresponding to the temperature of the heater 121. The value corresponding to the temperature of the heater 121 may be the temperature of the heater 121 itself or the resistance of the heater 121, which changes as the temperature of the heater 121 changes. For example, the controller 116 may perform the output control each time the temperature of the heater 121 increases by 100°C. With this configuration, the output of the heater 121 can reach the output target at an appropriate timing.

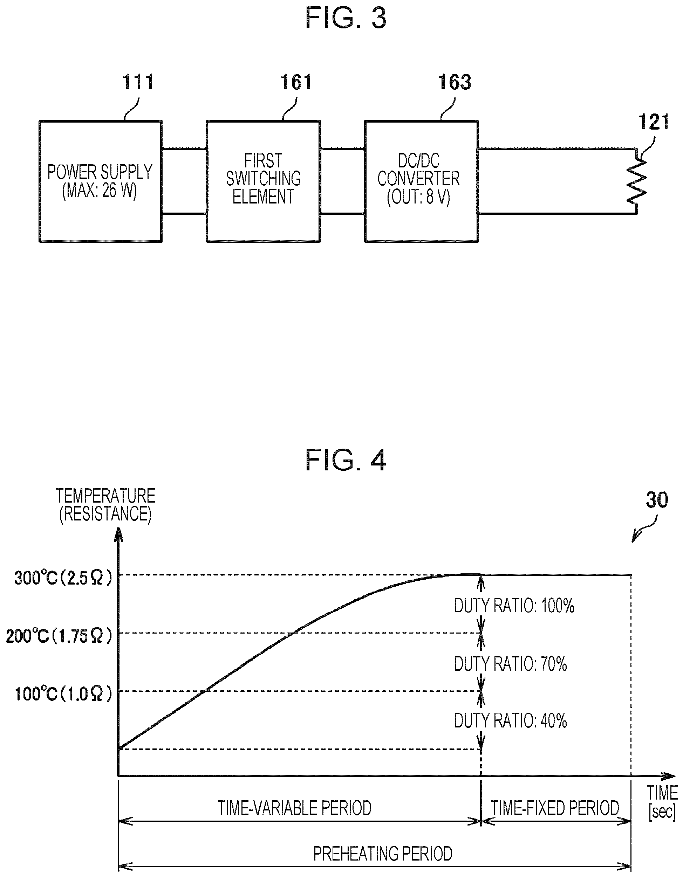

An example of the output control according to the present embodiment will be described in detail hereinafter with reference to Figs. 3 and 4.

Fig. 3 is a block diagram for describing the output control according to the present embodiment. Fig. 3 illustrates in detail an example of a circuit that connects the power supply 111 and the heater 121. As illustrated in Fig. 3, the inhaler device 100 includes a first switching element 161 and a DC (direct current)/DC converter 163 between the power supply 111 and the heater 121.

In the example illustrated in Fig. 3, the maximum output of the power supply 111 is 26 W.

The first switching element 161 is a device that starts and stops the supply of power to the heater 121. As the first switching element 161, a MOSFET (metal-oxide-semiconductor field-effect transistor), an IGBT (insulated gate bipolar transistor), a bipolar transistor, or the like can be employed. Starting the supply of power to the heater 121 will also be referred to as turning on the first switching element 161. Stopping the supply of power to the heater 121 will also be referred to as turning off the first switching element 161. In an example, the controller 116 may perform PWM control for the supply of power to the heater 121 using the first switching element 161. That is, the controller 116 may control the duty ratio by controlling a period of time for which the first switching element 161 is turned on. In another example, the controller 116 may perform PFM control for the supply of power to the heater 121 using the first switching element 161. That is, the controller 116 may control the duty ratio by controlling a frequency for turning on the first switching element 161.

The DC/DC converter 163 is a transformer that transforms a direct current voltage into another direct current voltage. As illustrated in Fig. 3, the DC/DC converter 163 is arranged between the power supply 111 and the heater 121. The DC/DC converter 163 transforms the voltage applied from the power supply 111 and applies a resultant voltage to the heater 121. The voltage input to the DC/DC converter 163 will also be referred to as an input voltage, and the voltage output from the DC/DC converter 163 will also be referred to as an output voltage. The input voltage and the output voltage are typically different from each other, but may be the same. The output voltage of the DC/DC converter 163 is applied to the heater 121. In the example illustrated in Fig. 3, the output voltage of the DC/DC converter 163 is 8 V, and 8 V is applied to the heater 121.

The heater 121 is a resistance heater. As described above, the resistance of the heater 121 (more specifically, the resistance heater constituting the heater 121) increases as the temperature of the heater 121 increases. The controller 116, therefore, controls, as the output control, the first switching element 161 such that a period of time for which power is supplied to the heater 121 in unit time increases as the temperature of the heater 121 increases. For example, the controller 116 increases the duty ratio of the power pulse supplied to the heater 121 as the temperature of the heater 121 increases. With this configuration, a decrease in the heating efficiency due to an increase in the resistance of the heater 121 can be cancelled by increasing the duty ratio of the power pulse. That is, the heating efficiency can be kept high. This point will be described with reference to Fig. 4.

Fig. 4 is a graph for describing the output control according to the present embodiment. A graph 30 of Fig. 4 indicates changes in the temperature of the heater 121 in the preheating period. A vertical axis of the graph 30 represents the temperature of the heater 121, and the resistance of the heater 121 is also shown. A horizontal axis of the graph 30 represents time (seconds).

As illustrated in Fig. 4, when the temperature of the heater 121 reaches 100°C, 200°C, and 300°C, the resistance of the heater 121 becomes 1.0 Ω, 1.75 Ω, and 2.5 Ω, respectively. The controller 116 sets the duty ratio to 40% in a period until the temperature of the heater 121 reaches 100°C. Next, the controller 116 sets the duty ratio to 70% in a period until the temperature of the heater 121 reaches 200°C after reaching 100°C. Next, the controller 116 sets the duty ratio to 100% in a period until the temperature of the heater 121 reaches 300°C after reaching 200°C. An output "P" of the heater 121 is calculated using the following expression when the resistance of the heater 121 is denoted by "R", the voltage applied to the heater 121 is denoted by "V", and the duty ratio is denoted by "D".

[Math. 1]

$$P = \frac{V^2}{R} \times D \qquad \cdots (1)$$

When the temperature of the heater 121 is 100°C, the output "P" of the heater 121 is 25.6 W according to the above Expression (1). When the temperature of the heater 121 is 200°C, the output "P" of the heater 121 is 25.6 W according to the above Expression (1). When the temperature of the heater 121 is 300°C, the output "P" of the heater 121 is 25.6 W according to the above Expression (1). The output "P" of the heater 121 is thus kept at the output target of 25.6 W, which is substantially the same as the maximum output of 26 W of the power supply 111. With this configuration, the preheating period can be shortened.

As a comparative example, an example is assumed in which the output voltage of the DC/DC converter 163 is 5 V and the duty ratio is constantly kept at 100%. When the temperature of the heater 121 is 100°C, the output "P" of the heater 121 is 25 W according to the above Expression (1). When the temperature of the heater 121 is 200°C, the output "P" of the heater 121 is 14.2 W according to the above Expression (1). When the temperature of the heater 121 is 300°C, the output "P" of the heater 121 is 10 W according to the above Expression (1). In the comparative example, the output "P" of the heater 121 thus decreases as the temperature of the heater 121 increases, and efficient heating becomes difficult. In the present embodiment, however, efficient heating can be achieved compared to the comparative example.

(3) Procedure of process

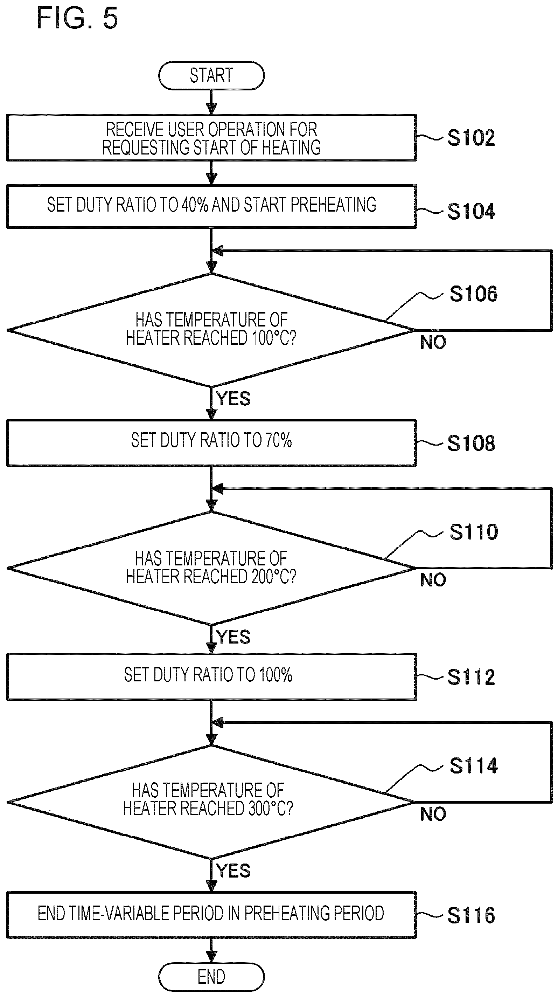

Fig. 5 is a flowchart illustrating an example of a procedure of a process performed by the inhaler device 100 according to the present embodiment.

As illustrated in Fig. 5, first, the inhaler device 100 receives a user operation for requesting a start of heating (step S102). An example of the user operation for requesting a start of heating is pressing of a button provided for the inhaler device 100. Another example of the user operation for requesting a start of heating is insertion of the stick substrate 150 into the container 140.

Next, the inhaler device 100 sets the duty ratio to 40% and starts the preheating (step S104).

Next, the inhaler device 100 determines whether the temperature of the heater 121 has reached 100°C (step S106). The inhaler device 100 waits until the temperature of the heater 121 reaches 100°C (step S106: NO).

If determining that the temperature of the heater 121 has reached 100°C (step S106: YES), the inhaler device 100 sets the duty ratio to 70% (step S108).

Next, the inhaler device 100 determines whether the temperature of the heater 121 has reached 200°C (step S110). The inhaler device 100 waits until the temperature of the heater 121 reaches 200°C (step S110: NO).

If determining that the temperature of the heater 121 has reached 200°C (step S110: YES), the inhaler device 100 sets the duty ratio to 100% (step S112).

Next, the inhaler device 100 determines whether the temperature of the heater 121 has reached 300°C (step S114). The inhaler device 100 waits until the temperature of the heater 121 reaches 300°C (step S114: NO).

If determining that the temperature of the heater 121 has reached 300°C (step S114: YES), the inhaler device 100 ends the time-variable period in the preheating period (step S116). The inhaler device 100 then performs the temperature control in, for example, a period in the preheating period subsequent to a period in which the time control is performed.

<3. Supplementary information>

Although a preferred embodiment of the present disclosure has been described in detail with reference to the accompanying drawings, the present disclosure is not limited to this example. It is clear that those who have ordinary knowledge in a technical field to which the present disclosure pertains can conceive various examples of alterations or modifications within the scope of the technical idea described in the claims, and it is understood that these also naturally belong to the technical scope of the present disclosure.

(1) First modification

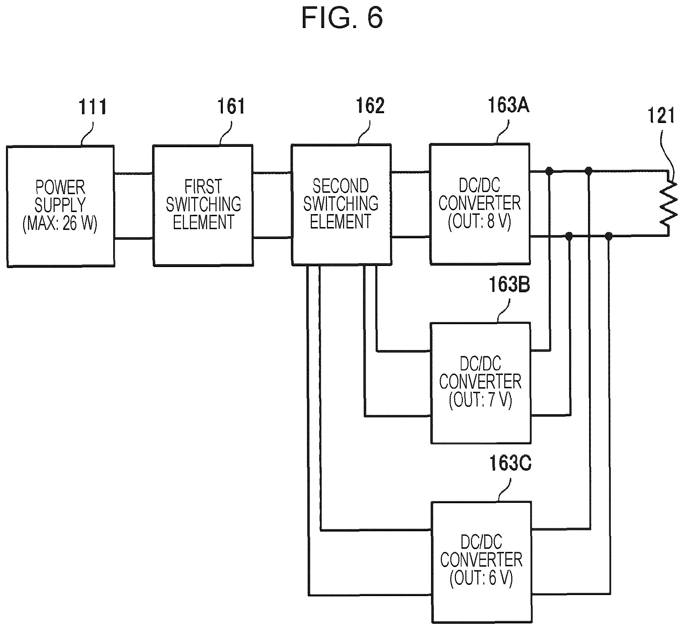

Although an example in which the inhaler device 100 controls, as the output control, the duty ratio of the power pulse supplied to the heater 121 has been described, the present disclosure is not limited to this example. The inhaler device 100 may switch, as the output control, the DC/DC converter 163 to be used. An example of the output control according to this modification will be described in detail hereinafter with reference to Fig. 6.

Fig. 6 is a block diagram for describing the output control according to the present modification. Fig. 6 illustrates in detail an example of a circuit that connects the power supply 111 and the heater 121. As illustrated in Fig. 6, the inhaler device 100 in the present modification includes the first switching element 161, a second switching element 162, and three DC/DC converters 163 (163A to 163C) between the power supply 111 and the heater 121.

In the example illustrated in Fig. 6, the maximum output of the power supply 111 is 26 W.

The configuration of the first switching element 161 is as described above with reference to Fig. 3. The controller 116, however, does not change the duty ratio as the output control. For example, the controller 116 may maintain the duty ratio at a constant value in the preheating period.

The configuration of the DC/DC converters 163 is as described above with reference to Fig. 3. Output voltages of the DC/DC converters 163A to 163C are different from one another. In the example illustrated in Fig. 6, the output voltage of the DC/DC converter 163A is 8 V. The output voltage of the DC/DC converter 163B is 7 V. The output voltage of the DC/DC converter 163C is 6 V.

The second switching element 162 switches one DC/DC converter 163 that applies a voltage to the heater 121 between the plurality of DC/DC converters 163. That is, the voltage output from one of the DC/DC converters 163A to 163C connected to the power supply 111 and the heater 121 by the second switching element 162 is applied to the heater 121. As the second switching element 162, a MOSFET (metal-oxide-semiconductor field-effect transistor), an IGBT (insulated gate bipolar transistor), a bipolar transistor, or the like can be employed. Switching performed by the second switching element 162 for applying the voltage output from the DC/DC converter 163A to the heater 121 will also be referred to as turning on the DC/DC converter 163A. The same holds for the DC/DC converters 163B and 163C.

The heater 121 is a resistance heater. As described above, the resistance of the heater 121 (more specifically, the resistance heater constituting the heater 121) increases as the temperature of the heater 121 increases. The controller 116, therefore, controls, as the output control, the second switching element 162 such that a DC/DC converter 163 whose output voltage is higher outputs a voltage to the heater 121 as the temperature of the heater 121 increases. For example, as the temperature of the heater 121 increases, the controller 116 switches the DC/DC converter 163 to be turned on from the DC/DC converter 163C to the DC/DC converter 163B and from the DC/DC converter 163B to the DC/DC converter 163A. With this configuration, a decrease in the heating efficiency due to an increase in the resistance of the heater 121 can be cancelled by turning on a DC/DC converter 163 whose output voltage is higher and increasing the voltage applied to the heater 121. That is, as in the above embodiment, heating efficiency can be kept high as in the above embodiment.

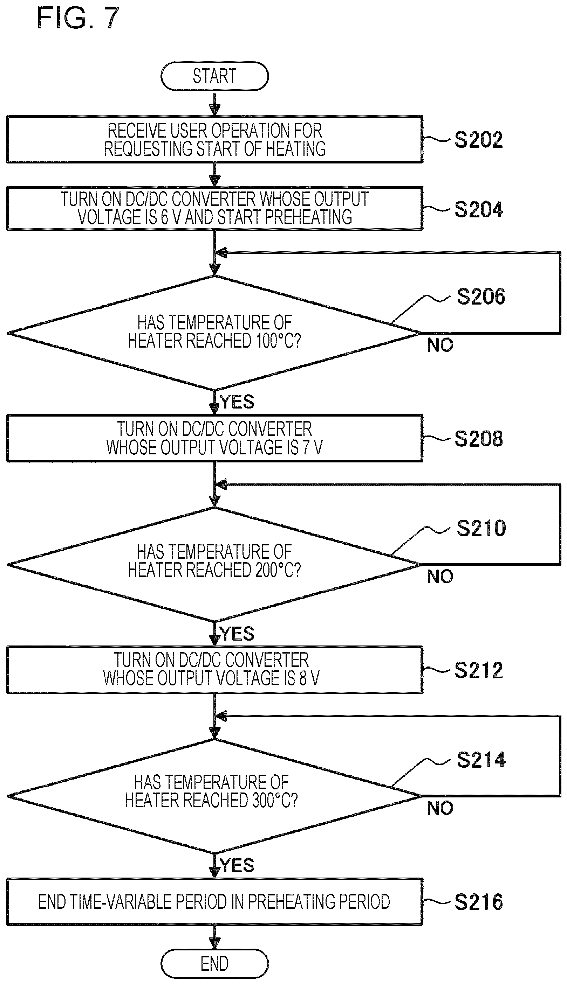

Next, a process performed by the inhaler device 100 according to the present modification will be described with reference to Fig. 7. Fig. 7 is a flowchart illustrating an example of a procedure of the process performed by the inhaler device 100 according to the present modification.

As illustrated in Fig. 7, first, the inhaler device 100 receives a user operation for requesting a start of heating (step S202).

Next, the inhaler device 100 starts the preheating by turning on the DC/DC converter 163C, whose output voltage is 6 V (step S204).

Next, the inhaler device 100 determines whether the temperature of the heater 121 has reached 100°C (step S206). The inhaler device 100 waits until the temperature of the heater 121 reaches 100°C (step S206: NO).

If determining that the temperature of the heater 121 has reached 100°C (step S206: YES), the inhaler device 100 turns on the DC/DC converter 163B, whose output voltage is 7 V (step S208).

Next, the inhaler device 100 determines whether the temperature of the heater 121 has reached 200°C (step S210). The inhaler device 100 waits until the temperature of the heater 121 reaches 200°C (step S210: NO).

If determining that the temperature of the heater 121 has reached 200°C (step S210: YES), the inhaler device 100 turns on the DC/DC converter 163A, whose output voltage is 8 V (step S212).

Next, the inhaler device 100 determines whether the temperature of the heater 121 has reached 300°C (step S214). The inhaler device 100 waits until the temperature of the heater 121 reaches 300°C (step S214: NO).

If determining that the temperature of the heater 121 has reached 300°C (step S214: YES), the inhaler device 100 ends the time-variable period in the preheating period (step S216). The inhaler device 100 then performs the temperature control in, for example, the period in the preheating period subsequent to the period in which the time control is performed.

(2) Second modification

The user sometimes puffs while sequentially attaching and removing a plurality of stick substrates 150 to and from the inhaler device 100 at short intervals and successively heating the stick substrates 150. Such a mode of use is called chain smoking. When chain smoking is performed, heating is started soon after an end of previous heating, and the temperature of the heater 121 is already high at a start of the heating. If no measures are taken, therefore, the preheating period, or more accurately the time-variable period in the preheating period, can be extremely short. When the preheating period is extremely short, the puffable period might start without moisture within the stick substrate 150 sufficiently evaporated, which deteriorates a smoke taste immediately after a start of the puffable period. When chain smoking is performed, therefore, measures need to be taken such that the preheating period does not become extremely short.

The controller 116, therefore, decreases the voltage applied to the heater 121 in unit time as the temperature of the heater 121 at a start of heating increases. In particular, the controller 116 decreases the voltage applied to the heater 121 in unit time in the preheating period as the temperature of the heater 121 at a start of heating increases. With this configuration, even when chain smoking is performed, it is possible to prevent the preheating period from becoming extremely short, thereby improving the smoke taste.

More specifically, with respect to the above embodiment, the controller 116 controls the first switching element 161 such that a period of time for which power is supplied to the heater 121 in unit time becomes shorter as the temperature of the heater 121 at a start of heating increases. In particular, the controller 116 controls the first switching element 161 such that the duty ratio in the preheating period decreases as the temperature of the heater 121 at a start of heating increases. When the temperature of the heater 121 is lower than 50°C at a start of heating, for example, the controller 116 sequentially changes the duty ratio from 40% to 70%, and then to 100%, as the temperature increases in the preheating period as described with reference to Fig. 4. When the temperature of the heater 121 is higher than or equal to 50°C at a start of heating, on the other hand, the controller 116 may sequentially change the duty ratio from 20% to 50%, and then to 80%, as the temperature increases in the preheating period. With this configuration, when chain smoking is performed, it is possible to prevent the preheating period from becoming extremely short, thereby improving the smoke taste.

In addition, as described in the first modification, the inhaler device 100 may include the plurality of DC/DC converters 163 whose output voltages are different from one another and the second switching element 162 that switches the DC/DC converter 163 to be turned on. In this case, the controller 116 controls the second switching element 162 such that a DC/DC converter 163 whose output voltage is lower applies a voltage to the heater 121 as the temperature of the heater 121 at a start of heating increases. In particular, the controller 116 controls the second switching element 162 such that a DC/DC converter 163 whose output voltage is lower applies a voltage to the heater 121 in the preheating period as the temperature of the heater 121 at a start of heating increases. When the temperature of the heater 121 is lower than 50°C at a start of heating, for example, the controller 116 may turn on the DC/DC converter 163A, whose output voltage is 8 V, in the preheating period. The controller 116 may then change the duty ratio from 40% to 70%, and then to 100%, as the temperature increases. When the temperature of the heater 121 is higher than or equal to 50°C at a start of heating, on the other hand, the controller 116 may turn on the DC/DC converter 163B, whose output voltage is 7 V, or the DC/DC converter 163C, whose output voltage is 6 V, in the preheating period. The controller 116 may then change the duty ratio from 40% to 70%, and then to 100%, as the temperature increases. With this configuration, when chain smoking is performed, it is possible to prevent the preheating period from becoming extremely short, thereby improving the smoke taste.

In other words, the controller 116 may decrease the output target as the temperature of the heater 121 at a start of heating increases. For example, when the temperature of the heater 121 is lower than 50°C at a start of heating, the controller 116 sets the output target to about 90% of the maximum output of the power supply 111. When the temperature of the heater 121 is higher than or equal to 50°C at a start of heating, on the other hand, the controller 116 may set the output target to about 70% of the maximum output of the power supply 111. With this configuration, when chain smoking is performed, it is possible to prevent the preheating period from becoming extremely short, thereby improving the smoke taste.

(3) Additional information

Although an example in which the output control is performed each time the temperature of the heater 121 increases by 100°C has been described above, the present disclosure is not limited to this example. Temperature intervals for performing the output control are not limited to 100°C, and any temperature intervals, such as 10°C or 1°C, may be set, instead. By reducing the temperature intervals for performing the output control, for example, changes in the duty ratio can be made closer to linear. As a result, it becomes possible to prevent the output of the heater 121 from deviating from the output target.

Two types of output control, namely control of the duty ratio and switching of the DC/DC converter 163 to be turned on, have been described above. The two types of control may be performed simultaneously.

Although an example in which the timing of performing the output control is controlled on the basis of the temperature of the heater 121 has been described, the present disclosure is not limited to this example. The controller 116 may control the timing of performing the output control on the basis of time elapsed since a start of heating, instead. For example, the controller 116 may perform the output control in cycles of 10 seconds. With this configuration, too, the output of the heater 121 can be brought to the output target at an appropriate timing. In addition, the time intervals of the output control are not limited to 10 seconds, and any time intervals, such as 5 seconds or 1 second, may be set, instead. By reducing the time intervals of the output control, for example, changes in the duty ratio can be made closer to linear. As a result, it becomes possible to prevent the output of the heater 121 from deviating from the output target.

In the above description, the output control is a process for increasing the voltage applied to the heater 121 in unit time as the temperature of the heater 121 increases. Alternatively, the output control may be regarded as a process for increasing an average of voltages applied to the heater 121 as the temperature of the heater 121 increases. Alternatively, the output control may be regarded as a process for increasing an effective value of the voltage applied to the heater 121 as the temperature of the heater 121 increases.

Although specific values of the maximum output of the power supply 111, the output voltages of the DC/DC converters 163, the resistance of the heater 121, and the like have been mentioned above, these are merely examples. Any other values may be employed, instead.

Although an example in which the parameter that is specified in the heating profile and that relates to the heating temperature of the aerosol source is the temperature of the heater 121 has been described above, the present disclosure is not limited to this example. The parameter relating to the heating temperature of the aerosol source may be the resistance of the heater 121, instead of the temperature of the heater 121 itself described in the above embodiment.

It is to be noted that the process by each device described herein may be achieved by software, hardware, or a combination of software and hardware. A program constituting software is stored in advance, for example, in a storage medium (more specifically, a non-transitory computer-readable storage medium) provided inside or outside each device. When executed by a computer that controls each device described herein, for example, each program is loaded into a RAM and executed by a processing circuit such as CPU. The storage medium is, for example, a magnetic disk, an optical disc, a magneto-optical disk, a flash memory, or the like. In addition, the computer program may be distributed over a network, instead, without using a storage medium. In addition, the computer may be an integrated circuit for a specific application such as an ASIC, a general-purpose processor that executes a function by reading a software program, a computer on a server used for cloud computing, or the like. In addition, the process by each device described herein may be performed by a plurality of computers in a distributed manner.

In addition, the process described herein with reference to the flowcharts and the sequence diagrams need not necessarily be performed in the illustrated order. Some processing steps may be performed in parallel with each other, instead. Additional processing steps may also be employed, or some processing steps may be omitted.

The following configurations also belong to the technical scope of the present disclosure.

(1) An aerosol generation system including:

a power supply;
a heater that heats an aerosol source included in a substrate using power supplied from the power supply; and
a controller that controls operation of the heater,
in which the controller performs output control for increasing a voltage applied to the heater in unit time as temperature of the heater increases.

(2) The aerosol generation system according to (1), further including:

a first switching element that starts or stops the supply of power to the heater,
in which the controller controls, as the output control, the first switching element such that a period of time for which power is supplied to the heater in unit time becomes longer as the temperature of the heater increases.

(3) The aerosol generation system according to (1), further including:

a plurality of transformers that is provided between the power supply and the heater and that transforms a voltage applied from the power supply and applies resultant voltages to the heater; and
a second switching element that switches the one transformer that applies a voltage to the heater between the plurality of transformers,
in which output voltages of the plurality of transformers are different from each other, and
in which the controller controls, as the output control, the second switching element such that the transformer whose output voltage is higher applies a voltage to the heater as the temperature of the heater increases.

(4) The aerosol generation system according to any of (1) to (3),
in which the controller performs the output control in a period after a start of the heating in which the temperature of the heater continues to increase.
(5) The aerosol generation system according to any of (1) to (4),

in which the heater is a resistance heater whose resistance changes in accordance with the temperature thereof, and
in which the controller performs the output control such that an output of the heater becomes a predetermined target value.

(6) The aerosol generation system according to (5),
in which the controller sets the target value on a basis of a maximum output of the power supply.
(7) The aerosol generation system according to any of (1) to (6),
in which the controller decreases the voltage applied to the heater in unit time as the temperature of the heater at a start of the heating increases.
(8) The aerosol generation system according to (2),
in which the controller controls the first switching element such that the period of time for which power is supplied to the heater in unit time becomes shorter as the temperature of the heater at a start of the heating increases.

(9) The aerosol generation system according to (3),
in which the controller controls the second switching element such that the transformer whose output voltage is lower applies a voltage to the heater as the temperature of the heater at a start of heating increases.
(10) The aerosol generation system according to (6),
in which the controller decreases the target value as the temperature of the heater at a start of the heating increases.
(11) The aerosol generation system according to any of (1) to (10),
in which the controller controls a timing of performing the output control on a basis of a value corresponding to the temperature of the heater.
(12) The aerosol generation system according to any of (1) to (10),
in which the controller controls a timing of performing the output control on a basis of time elapsed since a start of the heating.
(13) The aerosol generation system according to any of (1) to (12), further including:
the substrate.
(14) A control method executed by a computer that controls an inhaler device, the inhaler device including:

a power supply; and
a heater that heats an aerosol source included in a substrate using power supplied from the power supply,
the control method including:
performing output control for increasing a voltage applied to the heater in unit time as temperature of the heater increases.

(15) A program executed by a computer that controls an inhaler device, the inhaler device including:

a power supply; and
a heater that heats an aerosol source included in a substrate using power supplied from the power supply,
the program causing the computer to perform a process including:
performing output control for increasing a voltage applied to the heater in unit time as temperature of the heater increases.

Reference Signs List

100    inhaler device
111    power supply
112    sensor
113    notifier
114    memory
115    communicator
116    controller
121    heater
140    container
141    internal space
142    opening
143    bottom
144    heat insulator
150    stick substrate
151    substrate
152    inhalation port
161    first switching element
162    second switching element
163    DC/DC converter

**Claims**

1. An aerosol generation system comprising:

   a power supply;

a heater that heats an aerosol source included in a substrate using power supplied from the power supply; and
a controller that controls operation of the heater,
wherein the controller performs output control for increasing a voltage applied to the heater in unit time as temperature of the heater increases.

2. The aerosol generation system according to claim 1, further comprising:

a first switching element that starts or stops the supply of power to the heater,
wherein the controller controls, as the output control, the first switching element such that a period of time for which power is supplied to the heater in unit time becomes longer as the temperature of the heater increases.

3. The aerosol generation system according to claim 1, further comprising:

a plurality of transformers that is provided between the power supply and the heater and that transforms a voltage applied from the power supply and applies resultant voltages to the heater; and
a second switching element that switches the one transformer that applies a voltage to the heater between the plurality of transformers,
wherein output voltages of the plurality of transformers are different from each other, and
wherein the controller controls, as the output control, the second switching element such that the transformer whose output voltage is higher applies a voltage to the heater as the temperature of the heater increases.

4. The aerosol generation system according to any of claims 1 to 3,
wherein the controller performs the output control in a period after a start of the heating in which the temperature of the heater continues to increase.

5. The aerosol generation system according to any of claims 1 to 4,

wherein the heater is a resistance heater whose resistance changes in accordance with the temperature thereof, and
wherein the controller performs the output control such that an output of the heater becomes a predetermined target value.

6. The aerosol generation system according to claim 5,
wherein the controller sets the target value on a basis of a maximum output of the power supply.

7. The aerosol generation system according to any of claims 1 to 6,
wherein the controller decreases the voltage applied to the heater in unit time as the temperature of the heater at a start of the heating increases.

8. The aerosol generation system according to claim 2,
wherein the controller controls the first switching element such that the period of time for which power is supplied to the heater in unit time becomes shorter as the temperature of the heater at a start of the heating increases.

9. The aerosol generation system according to claim 3,
wherein the controller controls the second switching element such that the transformer whose output voltage is lower applies a voltage to the heater as the temperature of the heater at a start of heating increases.

10. The aerosol generation system according to claim 6,
wherein the controller decreases the target value as the temperature of the heater at a start of the heating increases.

11. The aerosol generation system according to any of claims 1 to 10,
wherein the controller controls a timing of performing the output control on a basis of a value corresponding to the temperature of the heater.

12. The aerosol generation system according to any of claims 1 to 10,
wherein the controller controls a timing of performing the output control on a basis of time elapsed since a start of the heating.

13. The aerosol generation system according to any of claims 1 to 12, further comprising:
    the substrate.

14. A control method executed by a computer that controls an inhaler device, the inhaler device including:

    a power supply; and
    a heater that heats an aerosol source included in a substrate using power supplied from the power supply,
    the control method comprising:
    performing output control for increasing a voltage applied to the heater in unit time as temperature of the heater
    increases.

15. A program executed by a computer that controls an inhaler device, the inhaler device including:

    a power supply; and
    a heater that heats an aerosol source included in a substrate using power supplied from the power supply,
    the program causing the computer to perform a process comprising:
    performing output control for increasing a voltage applied to the heater in unit time as temperature of the heater
    increases.

# FIG. 1

FIG. 2

# FIG. 3

# FIG. 4

FIG. 5

```
                    ┌─────────────┐
                    │    START    │
                    └──────┬──────┘
                           │
              ┌────────────▼──────────────┐
              │  RECEIVE USER OPERATION FOR │──── S102
              │ REQUESTING START OF HEATING │
              └────────────┬──────────────┘
                           │
              ┌────────────▼──────────────┐
              │ SET DUTY RATIO TO 40% AND  │──── S104
              │      START PREHEATING      │
              └────────────┬──────────────┘
                           │
                           ▼◄──────────────────────────┐
                          ╱ ╲                S106        │
                        ╱     ╲                          │
                      ╱  HAS TEMPERATURE OF ╲   NO       │
                      ╲  HEATER REACHED 100°C?╱──────────┘
                        ╲     ╱
                          ╲ ╱
                           │ YES
              ┌────────────▼──────────────┐
              │    SET DUTY RATIO TO 70%   │──── S108
              └────────────┬──────────────┘
                           │
                           ▼◄──────────────────────────┐
                          ╱ ╲                S110        │
                        ╱     ╲                          │
                      ╱  HAS TEMPERATURE OF ╲   NO       │
                      ╲  HEATER REACHED 200°C?╱──────────┘
                        ╲     ╱
                          ╲ ╱
                           │ YES
              ┌────────────▼──────────────┐
              │   SET DUTY RATIO TO 100%   │──── S112
              └────────────┬──────────────┘
                           │
                           ▼◄──────────────────────────┐
                          ╱ ╲                S114        │
                        ╱     ╲                          │
                      ╱  HAS TEMPERATURE OF ╲   NO       │
                      ╲  HEATER REACHED 300°C?╱──────────┘
                        ╲     ╱
                          ╲ ╱
                           │ YES
              ┌────────────▼──────────────┐
              │ END TIME-VARIABLE PERIOD   │──── S116
              │   IN PREHEATING PERIOD     │
              └────────────┬──────────────┘
                           │
                    ┌──────▼──────┐
                    │     END     │
                    └─────────────┘
```

# FIG. 6

| 111 | 161 | 162 | 163A | 121 |
|-----|-----|-----|------|-----|
| POWER SUPPLY (MAX: 26 W) | FIRST SWITCHING ELEMENT | SECOND SWITCHING ELEMENT | DC/DC CONVERTER (OUT: 8 V) | |

163B
DC/DC CONVERTER (OUT: 7 V)

163C
DC/DC CONVERTER (OUT: 6 V)

# FIG. 7

```
                    ┌─────────────┐
                    │    START    │
                    └──────┬──────┘
                           │
        ┌──────────────────▼──────────────────┐
        │  RECEIVE USER OPERATION FOR          │──── S202
        │  REQUESTING START OF HEATING         │
        └──────────────────┬──────────────────┘
                           │
        ┌──────────────────▼──────────────────┐
        │  TURN ON DC/DC CONVERTER WHOSE OUTPUT│──── S204
        │  VOLTAGE IS 6 V AND START PREHEATING │
        └──────────────────┬──────────────────┘
                           │
                  ┌────────▼────────┐
                  │ HAS TEMPERATURE │──── S206
                  │ OF HEATER       │ NO ──┐
                  │ REACHED 100°C?  │      │
                  └────────┬────────┘      │
                           │ YES           │
        ┌──────────────────▼──────────────────┐
        │  TURN ON DC/DC CONVERTER             │──── S208
        │  WHOSE OUTPUT VOLTAGE IS 7 V         │
        └──────────────────┬──────────────────┘
                           │
                  ┌────────▼────────┐
                  │ HAS TEMPERATURE │──── S210
                  │ OF HEATER       │ NO ──┐
                  │ REACHED 200°C?  │      │
                  └────────┬────────┘      │
                           │ YES           │
        ┌──────────────────▼──────────────────┐
        │  TURN ON DC/DC CONVERTER             │──── S212
        │  WHOSE OUTPUT VOLTAGE IS 8 V         │
        └──────────────────┬──────────────────┘
                           │
                  ┌────────▼────────┐
                  │ HAS TEMPERATURE │──── S214
                  │ OF HEATER       │ NO ──┐
                  │ REACHED 300°C?  │      │
                  └────────┬────────┘      │
                           │ YES           │
        ┌──────────────────▼──────────────────┐
        │ END TIME-VARIABLE PERIOD IN          │──── S216
        │ PREHEATING PERIOD                    │
        └──────────────────┬──────────────────┘
                           │
                    ┌──────▼──────┐
                    │     END     │
                    └─────────────┘
```

## INTERNATIONAL SEARCH REPORT

| International application No. |
|---|
| **PCT/JP2022/029039** |

| **A.** | **CLASSIFICATION OF SUBJECT MATTER** |
|---|---|

*A24F 40/57*(2020.01)i
FI: A24F40/57

According to International Patent Classification (IPC) or to both national classification and IPC

| **B.** | **FIELDS SEARCHED** |
|---|---|

Minimum documentation searched (classification system followed by classification symbols)

A24F40/57; A61M15/06; H05B3/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| Y | JP 2018-505696 A (JOYETECH (CHANGZHOU) ELECTRONICS CO., LTD. ) 01 March 2018 (2018-03-01) paragraphs [0091]-[0100], [0119]-[0121], fig. 9-11, 17 | 1-15 |
| Y | JP 2005-108690 A (KYOCERA CORP) 21 April 2005 (2005-04-21) paragraph [0020] | 1-15 |
| Y | JP 2007-301975 A (CANON INC) 22 November 2007 (2007-11-22) paragraphs [0018]-[0019], fig. 3 | 3, 9 |

☐ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 September 2022** | **04 October 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2022/029039**

| Patent document cited in search report | | | Publication date (day/month/year) | Patent family member(s) | | | Publication date (day/month/year) |
|---|---|---|---|---|---|---|---|
| JP | 2018-505696 | A | 01 March 2018 | US | 2017/0280779 | A1 | |
| | | | | paragraphs [0178]-[0199], [0257]-[0259], fig. 9-11, 17 | | | |
| | | | | US | 2019/0246699 | A1 | |
| | | | | WO | 2016/115890 | A1 | |
| | | | | WO | 2016/115891 | A1 | |
| | | | | WO | 2016/115892 | A1 | |
| | | | | WO | 2016/115893 | A1 | |
| | | | | EP | 3249488 | A1 | |
| | | | | CN | 104571190 | A | |
| | | | | CN | 104571191 | A | |
| | | | | CN | 104571192 | A | |
| | | | | CN | 104731127 | A | |
| | | | | KR | 10-2017-0107518 | A | |
| JP | 2005-108690 | A | 21 April 2005 | (Family: none) | | | |
| JP | 2007-301975 | A | 22 November 2007 | US | 2007/0235026 | A1 | |
| | | | | paragraphs [0027]-[0028], fig. 3 | | | |

Form PCT/ISA/210 (patent family annex) (January 2015)

23

**EP 4 563 024 A1**

**Patent documents cited in the description**

- JP 6930689 B